# EUROPÄISCHE PATENTANMELDUNG

(11) **EP 4 325 198 A1**
(43) Veröffentlichungstag der Anmeldung: **21.02.2024**
(21) Anmeldenummer: 23182737.9
(22) Anmeldetag: 30.06.2023
(51) Int. Cl.: G01N 1/28, G01N 1/02, C12M 1/00, C12M 1/12, B25J 21/00

(54) **BARRIERESYSTEM MIT HANDHABUNGSEINRICHTUNG ZUR AUTOMATISIERTEN PROBENNAHME UND VERFAHREN ZUR AUTOMATISIERTEN PROBENNAHME**

(30) Priorität: 16.08.2022 DE 102022120677
(71) Anmelder: Syntegon Technology GmbH, 71332 Waiblingen (DE)
(72) Erfinder: Ilgenfritz, Markus, 91555 Feuchtwangen (DE); Krauß, Ulrich, 74532 Ilshofen (DE); Nagler, Stefan, 73485 Unterschneidheim (DE); Stegmeier, Samuel, 74594 Kreßberg (DE)
(74) Vertreter: DREISS Patentanwälte PartG mbB

(57) **Zusammenfassung**

Die Erfindung betrifft ein Barrieresystem einer, insbesondere pharmazeutischen, Produktionsanlage, mit einem geschlossenen oder getrennten Bereich, in dem eine sterile Umgebung bereitgestellt wird, wobei in dem geschlossenen oder getrennten Bereich eine Handhabungseinrichtung zur automatisierten Probennahme mit einer Abklatschprobe angeordnet ist sowie Verfahren zur automatisierten Probennahme.

## Beschreibung

Beim mikrobiologischen Monitoring in z.B. einer pharmazeutischen Produktionsanlage kommen häufig Petrischalen und Abklatschproben zum Einsatz. Dabei handelt es sich um mit Nährmedium (bspw. Agar) gefüllte Kunststoffschalen mit einem Deckel. Petrischalen dienen dem Monitoring der Luft und Abklatschproben werden für das Monitoring von Oberflächen verwendet.

Um eine Probenentnahme auf Oberflächen zu ermöglichen sind die Abklatschproben dabei so ausgeführt, dass das Nährmedium leicht über den Rand der Kunststoffschale (im Folgenden als Boden bezeichnet) übersteht. Die beiden mikrobiologischen Proben (Petrischalen und Abklatschproben) unterscheiden sich in der Größe und Form, werden jedoch beide meist in 10er-Stapeln dreifach steril in Folienschlauchbeutel verpackt angeliefert.

Die Beutel mit den mikrobiologischen Proben werden zum Einsatz in eine pharmazeutische Produktionsanlage eingebracht, und dort über Handschuheingriffe oder automatisiert ausgepackt. Abschließend stehen sie für das Monitoring z.B. in einem Magazin bereit.

Abklatschproben werden im Rahmen der "Guten Herstellungspraxis" in pharmazeutischen Produktionsanlagen meist nach Produktionsende durchgeführt, um auf Oberflächen potentiell vorhandene Mikroorganismen (wie z.B. Pilze oder Bakterien) aufzunehmen und diese anschließend in einem Brutschrank ausbrüten zu können.

Bei den aktuell bekannten pharmazeutischen Produktionsanlagen wird dieser Vorgang nach Produktionsende manuell durch das Bedienpersonal durchgeführt. Dies erfolgt z.B. bei einer Produktionsanlage mit einem Barrieresystem, welches z.B. als Isolator ausgeführt sein kann, über einen Bediener, der über Handschuheingriffe in die Anlage eingreift. Der Bediener entnimmt eine unbenutzte Abklatschprobe aus z.B. einem Magazin. Der Bediener nimmt den Deckel der Probe ab und führt die Probennahme durch Aufdrücken der Schale auf die zu testende Oberfläche durch. Anschließend setzt der Bediener den Deckel wieder auf die Schale auf und beschriftet die Abklatschprobe (z.B. wann die Probe an welcher Stelle in der Anlage genommen wurde). Üblicherweise wird die Probe abschließend wieder in das Magazin zurückstellt.

Der Dokumentation der Stelle der Probenentnahme kommt dabei eine große Bedeutung zu, da mit den Abklatschproben auch die Wirksamkeit von Desinfektions- und Dekontaminationsmaßnahmen, wie z.B. dem Dekontaminationszyklus mit Wasserstoffperoxid (H2O2), an kritischen Produktionsbereichen nachgewiesen wird. Nur wenn lückenlos und fehlerfrei dokumentiert wird, an welcher Stelle welche Probe genommen wurde, können beim Nachweis von Mikroorganismen durch das Ausbrüten z.B. Chargen freigegeben oder auch gesperrt werden bzw. Dekontaminations- und Desinfektionsmaßnahmen gezielt verbessert werden.

Nachdem an allen vorgesehenen Stellen die Abklatschproben durchgeführt wurden, kann die Produktionsanlage geöffnet werden, das Magazin mit den mikrobiologischen Proben entnommen werden und die mikrobiologischen Proben in einem Labor ausgebrütet werden.

Es ist möglich die mikrobiologischen Proben über z.B. ein RTP-Transfersystem mit Doppeltüre (sog. Rapid-Transfer-Ports, bestehend aus Alpha-Ports und Beta-Container) aus der Produktionsmaschine zu entnehmen, so dass diese nicht durch einen potentiell unsterilen Raum transportiert werden müssen. Da die Proben durch einen Deckel verschlossen sind, werden sie in der Praxis jedoch meist bei geöffneten Türen aus den Anlagen entnommen und in ein Labor verbracht.

Die bisherigen Lösungen haben den Nachteil, dass für die Probennahme auf Oberflächen ein Eingriff in die Produktionsanlage durch einen Bediener über Handschuhe notwendig ist. Die Handschuheingriffe stellen jedoch ein mögliches Kontaminationsrisiko für die Maschine und somit auch für das zu verarbeitende Produkt dar. Weiterhin besteht durch den Handschuheingriff die Gefahr, dass eben durch diesen Bedienereingriff beim Nehmen der Probe eine Kontamination erfolgt und somit fälschlicherweise angenommen wird, dass die Anlage während der vorausgegangenen Produktion kontaminiert war. Deshalb ist es wünschenswert bei modernen pharmazeutischen Produktionsanlagen mit Barrieresystem auf Handschuheingriffe zu verzichten. Man spricht in diesem Fall dann von einem handschuhlosen Barrieresystem bzw. "gloveless isolator". Ohne Handschuheingriffe ist eine Probennahme auf Oberflächen durch den Bediener jedoch nicht mehr möglich. Ein Öffnen der Türen vor der Probennahme auf den Oberflächen ist ebenfalls nicht möglich, da dann ggf. von außen eine Kontamination der Anlage verursacht wird, welche während der Produktion noch nicht vorhanden war.

Weiterhin haben die bisherigen Lösungen (mittels Bediener und über Handschuheingriffe) zur Durchführung von Abklatschproben den Nachteil, dass diese nicht reproduzierbar sind, da die Probennahme auf kritischen Oberflächen von einem Bediener ausgeführt wird und dieser die Probe nicht immer an der exakt gleichen Stelle nimmt bzw. die Abklatschschale auch nicht immer mit exakt der gleichen Kraft die gleiche Zeit an die Oberfläche andrückt. Die Bedingungen der Probenentnahme variieren daher von Probe zu Probe.

Des Weiteren besteht die Gefahr, dass bei der Dokumentation der Stelle der Probennahme Fehler durch den Bediener geschehen und eine Zuordnung der einzelnen Proben zu den Orten der Probennahme später nicht mehr möglich ist.

Der vorliegenden Erfindung liegt die Aufgabe zugrunde, die Zuverlässigkeit der Probennahme auf Oberflächen von pharmazeutischen Produktionsanlagen zu verbessern.

Die vorliegende Erfindung ermöglicht eine vollautomatisierte und reproduzierbare Probennahme auf Oberflächen von pharmazeutischen Produktionsanlagen ohne einen Eingriff durch das Bedienpersonal. In klassischen Anlagen mit Barrieresystemen mit Handschuheingriffen können mit der Erfindung die Handhabungsschritte durch den Bediener minimiert werden und die Abklatschproben reproduzierbar durchgeführt und fehlerfrei dokumentiert werden.

Die Erfindung sieht hierzu ein Barrieresystem vor. Dieses Barrieresystem kann insbesondere ein Isolator sein. Das Barrieresystem ist Teil einer Produktionsanlage oder für eine solche vorgesehen. Die Produktionsanlage ist insbesondere eine pharmazeutische Produktionsanlage. Das Barrieresystem kann auch ein "Restricted Access Barrier System" (RABS) sein. RABS trennen Produkte und Prozesse physisch von der Produktionsumgebung und dem Anwender und gewährleisten so einen hohen Produkt- und Bedienerschutz. Dabei handelt es sich typischerweise um eine Schutzverkleidung mit Glasscheiben, wobei in dem getrennten Bereich eine gerichtete Luftströmung vorherrscht, der getrennte Bereich ist aber nicht ganz geschlossen, sondern die Luft kann nach unten (bspw. knapp oberhalb der Tischplatte) nach außen abströmen.

Das Barrieresystem kann geschlossen mit einem geschlossenen Bereich ausgeführt sein, in dem eine sterile Umgebung bereitgestellt wird. Das Barrieresystem kann auch offen (RABS) mit einem abgetrennten Bereich sein, der zwar nicht vollständig geschlossen ist aber in dem eine sterile Umgebung (der Grad der Sterilität ist hier typischerweise geringer als in einem System mit geschlossenen Bereich, im Folgenden wird aber in beiden Fällen von einer sterilen Umgebung gesprochen) bereitgestellt wird.

Erfindungsgemäß ist nun vorgesehen, dass in dem geschlossenen oder getrennten Bereich eine Handhabungseinrichtung und eine Abklatschprobenaufnahme angeordnet sind. Die Abklatschprobenaufnahme ist ausgebildet, um wenigstens eine Abklatschprobe bereitzustellen. Die Handhabungseinrichtung ist ausgebildet, um die bereitgestellte Abklatschprobe aus der Abklatschprobenaufnahme automatisiert zu entnehmen, zu öffnen und eine Probennahme auf einem Oberflächenabschnitt in dem geschlossenen oder getrennten Bereich reproduzierbare durchzuführen. Mit reproduzierbar ist damit gemeint, dass die Handhabungseinrichtung in der Lage ist einen Probennahmevorgang mit gleichen Probennahmeparamtern zu wiederholen bspw. mit gleicher Andrückdauer und gleichem Andrückdruck der Probe. Die Probennahmeparameter sind dabei insbesondere gespeichert und/oder auslesbar. Die Handhabungseinrichtung ist dabei ausgebildet um die Schritte automatisiert auszuführen, das heißt ohne, dass ein menschlicher Eingriff bspw. über Handschuheingriffe nötig ist oder es kann auch vorgesehen sein, das die Probennahme ohne eine direkte Steuerung, sondern im Rahmen eines vorher festgelegten Ablaufs erfolgt. Bspw. kann ein Roboterarm verwendet werden. Insbesondere kann die Handhabungseinrichtung mehrere Probeentnahmestellen mit Abklatschproben beproben.

Eine Abklatschprobe im Sinne der vorliegenden Erfindung umfasst einen Boden auf dem ein Nährmedium, insbesondere Agar, angeordnet ist. Die Abklatschprobe ist im Ausgangszustand mit einem Deckel verschlossen.

Das Öffnen der Abklatschprobe erfolgt durch Abnehmen des Deckels vom Boden der Abklatschprobe.

Die Probennahme erfolgt durch Kontaktieren einer zu beprobenden Stelle mit dem Nährmedium der Abklatschprobe. Insbesondere ist vorgesehen, dass die Abklatschprobe vor dem Beproben mit dem Nährmedium nach unten und dem Boden nach oben ausgerichtet wird (mittels der Handhabungseinrichtung) und dann insbesondere mit einer Bewegung senkrecht zur Oberfläche des Nährmediums auf die zu beprobende Oberfläche bewegt wird. Insbesondere wird die zu beprobende Oberfläche mit einem vorbestimmten Druck und/oder für eine bestimmte Dauer mit dem Nährmedium in Kontakt gebracht.

Ggf. vorhandene Keime die an der zu beprobenden Oberfläche vorhanden sind werden so, ohne dass ein Bediener in den geschlossenen oder getrennten Bereich eingreifen muss, auf das Nährmedium aufgebracht und können erkannt werden. Eine Kontamination der sterilen Umgebung kann so erkannt werden.

Insbesondere ein kontrolliertes Beproben (genauer Druck und genaue Dauer des Andrückens) wird durch die Erfindung ermöglicht.

Weiter kann vorgesehen sein, dass die Handhabungseinrichtung ausgebildet ist, um die Abklatschprobe bei der Probennahme direkt zu greifen. Dies stellt eine besonders einfache Art der Beprobung dar und minimiert den konstruktiven Aufwand.

Alternativ kann weiter vorgesehen sein, dass die Handhabungseinrichtung ausgebildet ist, um die Abklatschprobe nach dem Entnehmen aus der Abklatschprobenaufnahme, welche z.B. als Magazin ausgebildet sein kann, einem Probenträger zuzuführen und bei der Probennahme den Probenträger zu bewegen. Der Probenträger kann insbesondere autoklavierbar ausgebildet sein. Im Gegensatz zu den Handschuheingriffen, welche bei einer manuellen Probennahme notwendig sind und welche nur mit Wasserstoffperoxid (H2O2) dekontaminiert werden verringert das Autoklavieren der Vorrichtung das Risiko einer Kontamination der Anlage, des Produkts bzw. ein Verfälschen der Probe.

Der Probenträger hat dabei die Aufgabe die Abklatschprobe sicher aufzunehmen, zu halten und ein Andrücken der Abklatschprobe auf eine Oberfläche zu vereinfachen. Insbesondere kann die Abklatschprobe mit ihrem Boden flächig auf dem Probenträger aufliegen, bzw. der Probenträger eine entsprechende Anlagefläche umfassen. Hierdurch kann effizient verhindert werden, dass die Abklatschprobe bzw. deren Boden beim Andrücken an die zu beprobende Oberfläche beschädigt wird.

Das Halten der Abklatschprobe an dem Probenträger kann kraft- oder formschlüssig oder durch eine Kombination aus Kraft- und Formschluss erfolgen. Der Kraftschluss zwischen der Vorrichtung und der Abklatschprobe kann beispielsweise durch eine mikrostrukturierte Polymerfolie oder auch durch einen Vakuumsauger erfolgen. Formschluss kann bspw. mittels Greiffinger an dem Probenträger erreicht werden. Die Greiffinger können aufweitbar und mit einer Ausnehmung ausgebildet sein, in die die Abklatschprobe einrasten kann.

Die Finger können umfänglich verteilt um die Anlagefläche verteilt angeordnet sein. Die Anlagefläche kann mit der eben erwähnten mikrostrukturierten Polymerfolie bezogen sein. Die Anlagefläche kann eine Ansaugöffnung des Vakuumsaugers umfassen. Der Anschluss für eine Vakuumleitung kann auf einer der Anlagefläche gegenüberliegend angeordneten Seite des Probenträger angeordnet sein.

Die Vorrichtung kann weiter eine Deckelhandhabungseinrichtung umfassen. Die Deckelhandhabungseinrichtung kann eingerichtet sein, um den Deckel einer Probe, die durch die Handhabungseinrichtung gehalten ist, oder auf dem Probenträger angeordnet ist, abzunehmen und/oder aufzusetzen. Die Deckelhandhabungseinrichtung kann als Pneumatikzylinder ausgebildet sein.

Es kann weiter vorgesehen sein, dass die Handhabungseinrichtung einen Greifer mit zwei Greifbacken, die aufeinander zu und voneinander weg bewegbar sind, umfasst. Dabei kann der Greifer an einem um wenigstens 90°, insbesondere um 180°, insbesondere um 360°, schwenkbaren Greiferglied der Handhabungseinrichtung angeordnet sein. Hierdurch ist der Greifer besonderes flexibel einsetzbar. Die Greiferbacken können insbesondere komplementär zu den Abklatschproben (gekrümmt, da diese üblicherweise kreisrund ausgebildet sind) ausgebildet sein.

Die Greifbacken der Handhabungseinrichtung können insbesondere derart ausgebildet sein, dass sie Abschnitte umfassen, die komplementär zu der runden Form der Abklatschproben ausgebildet sind und/oder Abschnitte umfassen, die komplementär zu der Form eines greifbaren Abschnitts des Probenträgers ausgebildet sind. Der Probenträger und die Handhabungseinrichtung können auch eine Kopplungseinrichtung aufweisen mittels der der Probenträger in einer definierten Position und Ausrichtung mit der Handhabungseinrichtung lösbar verbindbar ist. Dies kann beispielsweise durch einen formschlüssigen Hintergriff realisiert sein. Der Probenträger kann eine schlitzartige Ausnehmung umfassen in die eine komplementäre Kopplungseinrichtung der Handhabungseinrichtung eingebracht werden kann, wobei die Kopplungseinrichtung der Handhabungseinrichtung die schlitzartige Ausnehmung im gekoppelten Zustand hintergreifen kann.

Es kann weiter vorgesehen sein, dass die Abklatschprobenaufnahme eine Halterung für den Probenträger umfasst. Hierdurch ist der Probenträger nahe an Abklatschprobenaufnahme angeordnet und in einer definierten Position angeordnet. Die Handhabungseinrichtung kann eine Abklatschprobe aus der Abklatschprobenaufnahme entnehmen und auf dem Probenträger platzieren, insbesondere auf dessen Anlagefläche. Insbesondere kann die Abklatschprobe auf der Anlagefläche kraft- und/oder formschlüssig (wie oben beschrieben bspw. mittels Vakuum, mikrostrukturierter Polymerfolie und/oder Greiferfingern oder anderen Formschlusselementen, die die Abklatschprobe formschlüssig erfassen können) gehalten sein. Die Handhabungseinrichtung kann dann den Deckel der Abklatschprobe abnehmen. Die Handhabungseinrichtung kann dann den Probenträger oder die Abklatschprobe aufnehmen und die Beprobung der Oberfläche vornehmen.

Es kann weiter vorgesehen sein, dass die Abklatschprobenaufnahme zur Aufnahme mehrerer Abklatschproben ausgebildet ist und die Abklatschproben horizontal in der Abklatschprobenaufnahme anordenbar sind. Hierdurch sind die Abklatschproben einfach zugänglich und können zeitlich geordnet für die Probennahme verwendet werden.

Es kann weiter vorgesehen sein, dass das Barrieresystem ein Erfassungsgerät umfasst, das ausgebildet ist, um ein Identifizierungsmerkmals der Abklatschprobe zu erfassen, und insbesondere um die identifizierte Abklatschprobe der Zeit und/oder dem Ort einer Probennahme zuzuordnen. Es kann dabei weiter vorgesehen sein, dass das Erfassungsgerät einen Strichcodeleser, ein RFID-Lesegerät oder einen DataMatrixCode-Leser umfasst, der ausgebildet ist, um einen entsprechenden Code auf, in oder an einer Abklatschprobe zu lesen. Das Identifizierungsmerkmal kann also ein Strichcode oder DataMatrixCode sein oder ein RFID-Merkmal, das auslesbar an, in oder auf der Abklatschprobe angebracht ist, und das Erfassungsgerät kann ein entsprechendes Lesegerät umfassen bzw. sein. Hierdurch kann ein bestimmter Probenentnahmevorgang (Zeitpunkt und Ort der Probennahme) einer genau identifizierbaren Abklatschprobe über deren Identifizierungsmerkmal zugeordnet werden. Das ermöglicht eine hohe Nachvollziehbarkeit und Dokumentierbarkeit der Probennahme.

Die Abklatschprobe kann wie gesagt einen RFID-Chip umfassen. Auf dem RFID-Chip kann das Identifizierungsmerkmal bereitgestellt sein. Der RFID-Chip kann jedoch alternativ oder zusätzlich auch andere Informationen umfassen und/oder Informationen können auf dem RFID-Chip gespeichert werden.

Es kann weiter vorgesehen sein, dass das Erfassungsgerät ausgebildet ist, um den Ort und/oder den Zeitpunkt einer Probenentnahme zu erfassen. Dabei kann weiter vorgesehen sein, dass das Barrieresystem eine Datenverarbeitungsanlage umfasst, die eingerichtet ist, um den Ort und/oder den Zeitpunkt der Probenentnahme in einem Datensatz einer Abklatschprobe bzw. deren Identifizierungsmerkmal zuzuordnen und ggf. zu speichern und in einer Datenbank abzulegen.

Es kann weiter vorgesehen sein, dass das das Barrieresystem handschuheingrifffrei ausgebildet ist. Durch die Erfindung werden Handschuheingriffe überflüssig. Die Handschuheingriffe stellen Schwachstellen im Barrieresystem dar. Sie nicht zu verwenden erhöht die Zuverlässigkeit des Barrieresystems.

Wie bereits Eingangs ausgeführt, so betrifft die vorliegende Erfindung auch ein Verfahren zur automatisierten Probennahme in einem geschlossenen oder getrennten Bereich eines Barrieresystems. Das Barrieresystems ist insbesondere ein Isolator einer Produktionsanlage, die insbesondere eine pharmazeutische Produktionsanlage ist. In dem geschlossenen oder getrennten Bereich wird dabei eine sterile Umgebung bereitgestellt. Mit dem Verfahren kann zuverlässig die Qualität der sterilen Umgebung überwacht, dokumentiert und sichergestellt werden.

In dem geschlossenen oder getrennten Bereich ist wenigstens eine Handhabungseinrichtung angeordnet. Weiter ist in dem geschlossenen oder getrennten Bereich eine Abklatschprobe bereitgestellt.

Die Handhabungseinrichtung erfasst im Rahmen des erfindungsgemäßen Verfahrens die bereitgestellte Abklatschprobe. Die Handhabungseinrichtung öffnet die Abklatschprobe. Die Handhabungseinrichtung führt also einen Arbeitsschritt aus, in dem der Deckel der Abklatschprobe entfernt wird, so dass das Nährmedium der Abklatschprobe zugänglich ist. Die Handhabungseinrichtung führt mit der geöffneten Abklatschprobe automatisiert eine Probennahme auf einem Oberflächenabschnitt in dem geschlossenen oder getrennten Bereich durch. Bei der Probennahme wird die Abklatschprobe bzw. deren Nährmedium in Kontakt mit der zu beproben Oberfläche gebracht. Die Probennahme erfolgt dabei reproduzierbar. Die Probennahmeparamter sind also widerholbar. Eine zweite Probennahme kann mit gleichen Parametern durchgeführt werden.

Erfindungsgemäß ist insbesondere vorgesehen, dass nach einem ersten Produktionsintervall eine erste Beprobung mittels des eben beschriebenen Verfahrens oder den später beschriebenen Ausgestaltungen oder dem Alternativen Verfahren mit zwei Handhabungseinrichtungen erfolgt. Diese erste Beprobung erfolgt mit ersten Probennahmeparametern. Nach einem zweiten Produktionsintervall erfolgt eine zweite Beprobung mittels des eben beschriebenen Verfahrens oder den später beschriebenen Ausgestaltungen oder dem Alternativen Verfahren mit zwei Handhabungseinrichtungen erfolgt. Diese erste Beprobung erfolgt mit zweiten Probennahmeparametern. Die ersten und zweiten Probennahmeparameter können dabei wenigstens einen vorzugsweise mehrere identische Probennahmeparameter umfassen, vorzugsweise sind die Probennahmeparameter alle identisch. Die Probennahmeparameter können einen oder mehrere der folgenden umfassen: Anpressdruck der Probe, Anpressdauer der Probe, Ort der Probennahme.

Es kann erfindungsgemäß zur Durchführung des Verfahrens vorgesehen sein, dass die Handhabungseinrichtung die Abklatschprobe nach dem Erfassen auf einem Probenträger platziert. Der Probenträger hält die Abklatschprobe insbesondere kraft- und/oder formschlüssig. Nach dem platzieren auf dem Probenträger öffnet die Handhabungseinrichtung die Abklatschprobe. Zur Probennahme kann die Handhabungseinrichtung den Probenträger greifen und den Probenträger mit der an ihm gehaltenen Abklatschprobe an den zu beprobenden Oberflächenabschnitt in dem geschlossenen oder getrennten Bereich bewegen und die Probennahme durchführen. Hierzu wird die Abklatschprobe bzw. deren Nährmedium in Kontakt mit der zu beprobenden Oberfläche gebracht. Der Probenträger mit der darauf angeordneten Abklatschprobe wird dabei mittels der Handhabungseinrichtung bewegt.

Es kann weiter vorgesehen sein, dass die Handhabungseinrichtung bei der Probennahme die Abklatschprobe oder den Probenträger mit der an ihm gehaltenen Abklatschprobe derart orientiert, dass eine Probenoberfläche der Abklatschprobe parallel zur Oberfläche des beprobten Oberflächenabschnitts ausgerichtet ist. Die Abklatschprobe kann dann insbesondere mit definiertem Druck und/oder für eine definierte Dauer an diesen durch eine translatorische Bewegung angedrückt werden. Durch die parallele Orientierung wird die Bewegung zum Andrücken besonders einfach.

Es kann weiter vorgesehen sein, dass die Handhabungseinrichtung nach der Probennahme die Abklatschprobe wieder verschließt. Die Handhabungseinrichtung kann hierzu die Abklatschprobe oder den Probenträger mit der daran gehaltenen Abklatschprobe abstellen und den Deckel aufsetzen. Gegebenenfalls kann die Handhabungseinrichtung die Abklatschprobe oder den Probenträger mit der daran gehaltenen Abklatschprobe wieder aufnehmen und einem weiteren Arbeitsschritt zuführen. Beispielsweise kann die Abklatschprobe einem Lager für die Probe zugeführt werden oder einer Schleuse, um aus dem geschlossenen oder getrennten Bereich entnommen zu werden.

Es kann weiter vorgesehen sein, dass die Handhabungseinrichtung die Abklatschprobe vor oder nach der Probennahme einem Erfassungsgerät zuführt. Das Erfassungsgerät erfasst ein Identifizierungsmerkmal der Abklatschprobe. Das Identifizierungsmerkmal kann dann einer Probennahme, insbesondere deren Zeit und/oder Ort zugeordnet werden. Das Identifizierungsmerkmal kann ein Strichcode oder ein Datamatrix-Code sein (die Abklatschprobe kann auch einen RFID-Chip umfassen, der das Identifizierungsmerkmal auslesbar bereitstellt). Das Erfassungsgerät kann eine Kamera sein oder umfassen und/oder ein Lesegerät für einen Strichcode und/oder einen Datamatrix-Code oder ein RFID-Lesegerät. Insbesondere kann das Identifizierungsmerkmal in einer Datenverarbeitungsanlage gemeinsam mit Daten, die die Probennahme charakterisieren, insbesondere deren Zeit und/oder Ort (Entnahmestelle in dem geschlossenen oder getrennten Bereich), abgespeichert werden. Beispielsweise kann das Identifizierungsmerkmal in einer Tabelle gemeinsam mit den Daten, die die Probennahme charakterisieren abgespeichert werden. Dies begünstigt eine automatisierte Aufzeichnung der einzelnen Probennahmen.

Es kann weiter vorgesehen sein, dass die Handhabungseinrichtung die Abklatschprobe vor der Probennahme aus einer Abklatschprobenaufnahme entnimmt und/oder nach der Probennahme einer Abklatschprobenaufnahme zuführt. Die Abklatschprobenaufnahme kann regalartig ausgebildet sein mit Fächern oder Aufnahmen für eine Mehrzahl an Abklatschproben. Die Abklatschprobenaufnahme kann beispielsweise über eine Schleuse mit den darin befindlichen Proben in den geschlossenen oder getrennten Bereich einbringbar oder entnehmbar sein. Dazu kann die Abklatschprobenaufnahme lösbar im geschlossenen oder getrennten Bereich befestigt sein.

Wie bereits Eingangs ausgeführt, so betrifft die vorliegende Erfindung auch ein Verfahren zur automatisierten Probennahme in einem geschlossenen oder getrennten Bereich eines Barrieresystems wobei in dem geschlossenen oder getrennten Bereich wenigstens zwei Handhabungseinrichtungen angeordnet sind. Das Barrieresystem kann ein Isolator sein. Das Barrieresystem ist Teil einer Produktionsanlage, insbesondere einer pharmazeutischen Produktionsanlage. Der geschlossene Bereich stellt eine sterile Umgebung bereit. In dem geschlossenen oder getrennten Bereich ist eine Abklatschprobe bereitgestellt. Die bereits genannten Weiterbildungen bezogen auf das Verfahren mit einer Handhabungseinrichtung und bezogen auf das Barrieresystem sind auch als Weiterbildungen des Verfahrens mit wenigstens zwei Handhabungseinrichtungen zu verstehen.

Das Verfahren umfasst die Schritte:
Eine der Handhabungseinrichtungen erfasst die bereitgestellte Abklatschprobe. Eine der Handhabungseinrichtungen öffnet die bereitgestellte Abklatschprobe. Eine der Handhabungseinrichtung führt mit der Abklatschprobe eine Probennahme auf einem Oberflächenabschnitt in dem geschlossenen oder getrennten Bereich durch. Das Verfahren umfasst optional einen oder mehrere der nachfolgenden Schritte. Verschließen der Abklatschprobe nach der Probennahme. Platzieren der Abklatschprobe nach dem Erfassen auf einem Probenträger, der die Abklatschprobe, insbesondere kraft- und/oder formschlüssig, hält. Den Probenträger zur Probennahme greifen und den Probenträger mit der an ihm gehaltenen Abklatschprobe an einen Oberflächenabschnitt in dem geschlossenen oder getrennten Bereich bewegen und nachfolgend die Probennahme durchführen. Dabei sieht das Verfahren vor, das jede der Handhabungseinrichtungen wenigstens einen der Schritte des Verfahrens durchführt. Beispielsweise kann vorgesehen sei, dass eine erste Handhabungseinrichtung die Abklatschprobe aus einer Abklatschprobenaufnahme entnimmt, während eine zweite Handhabungseinrichtung den Probenträger aufnimmt, die erste Handhabungseinrichtung stellt die Abklatschprobe auf den Probenträger und nimmt den Deckel ab. Die zweite Handhabungseinrichtung führt dann mit der Probe, die durch den Probenträger gehalten ist, die Probennahme durch. Die erste Handhabungseinrichtung kann nach Durchführung der Probennahme den Deckel wieder aufsetzen und die Abklatschprobe vom Probenträger nehmen und wieder der Abklatschprobenaufnahme zuführen oder einer Schleuse des Barrieresystems.

Die arbeitsteilige Verwendung von zwei Handhabungseinrichtungen hat den Vorteil, dass Vorgänge schneller durchgeführt werden können und der Aktionsradius der Handhabungseinrichtungen größer als bei einer einzelnen Handhabungseinrichtung ist.

Im Sinne der vorliegenden Erfindung ist insbesondere, dass der Erfassungsbereich des Erfassungsgerät den Aktionsradius der Handhabungseinrichtung abdeckt oder dass die Handhabungseinrichtung eine Abklatschprobe dem Erfassungsbereich des Erfassungsgeräts zuführen kann. Die Dokumentation der Probennahme ist vorteilhaft, da hierdurch der Zustand des geschlossenen oder getrennten Bereichs sicher und zuverlässig protokolliert werden kann.

Die Handhabungseinrichtung kann die Abklatschprobe mittels beispielsweise eines Kraftsensors kraftgesteuert oder, beispielsweise mit Hilfe eines federnden Elements, weggesteuert immer mit der gleichen Kraft auf die Oberfläche aufdrücken. Die Reproduzierbarkeit der Probennahme kann damit nochmals erhöht werden.

Der Kraftsensor oder das federnde Element können in der Handhabungseinrichtung oder in dem Probenträger angeordnet sein. Ebenfalls im Sinne der Erfindung ist eine Kombination aus Kraft- und Wegsteuerung. Die dabei erzeugten Daten wie z.B. Anpresskraft und Zeit, können ebenfalls zur Dokumentation verwendet werden.

Ein Aufdrücken der Abklatschprobe mit einer definierten Kraft kann dazu beitragen, dass das Nährmedium, bspw. der Agar nicht aus der Probe fällt. Dies kommt bei der manuellen Probennahme durch Bediener gelegentlich vor und stört den Ablauf der Produktion bzw. der Probennahme, die üblicherweise nach der Produktion erfolgt.

Im Sinne der vorliegenden Erfindung ist insbesondere auch vorgesehen, dass Handhabungseinrichtung mehrere unterschiedlich im geschlossenen oder getrennten Bereich angeordnete Probennahmestellen beproben kann.

Das Erfassungsgerät oder eine weitere Überwachungseinrichtung können vorgesehen und ausgebildet sein, um im Anschluss an die Probennahme zu prüfen, ob das Nährmedium sich noch in der Abklatschprobe befindet. Die Überwachungseinrichtung kann beispielsweise als ein Kamerasystem ausgeführt sein. Wird die Abwesenheit des Nährmediums in der Probe festgestellt, so kann die Probennahme bspw. an einer vorab definierten weiteren Stelle wiederholt werden.

Erfindungsgemäß ist insbesondere vorgesehen, dass nach einem ersten Produktionsintervall eine erste Beprobung (Probennahme) mittels einem der eben beschriebenen Verfahren (Verfahren mit einer Handhabungseinrichtung oder Verfahren mit 2 Handhabungseinrichtungen) erfolgt. Diese erste Beprobung erfolgt mit ersten Probennahmeparametern. Probennahmeparameter sind dabei die Parameter, die die Probennahme charakterisieren. Anhand der Probennahmeparameter lässt sich die Art und Weise der Probennahme nachvollziehen. Nach einem zweiten Produktionsintervall erfolgt eine zweite Beprobung (Probennahme) mittels einem der eben beschriebenen Verfahren (Verfahren mit einer Handhabungseinrichtung oder Verfahren mit 2 Handhabungseinrichtungen). Diese zweite Beprobung erfolgt mit zweiten Probennahmeparametern. Die ersten und zweiten Probennahmeparameter umfassen dabei wenigstens einen vorzugsweise mehrere identische Probennahmeparameter, vorzugsweise sind die Probennahmeparameter alle identisch. Die Probennahmeparameter können einen oder mehrere der folgenden Parameter umfassen: Anpressdruck der Probe, Anpressdauer der Probe, Ort der Probennahme.

Das erste Produktionsintervall und das zweite Produktionsintervall können insbesondere gleich lang sein. Die erste und zweite Probennahme können insbesondere nach gleich langer Betriebsdauer erfolgen.

Die Probennahmeparameter können insbesondere auf einem RFID-Chip der jeweiligen verwendeten Abklatschprobe gespeichert werden und aus diesem auslesbar sein.

Nachfolgend wird die Erfindung im Detail anhand der beigefügten Figuren näher erläutert. Es zeigen:
- Fig. 1: eine schematisch dargestellte Produktionsanlage mit einem geschlossenen oder getrennten Bereich;
- Fig. 2: eine Abklatschprobe in einer Schnittansicht;
- Fig. 3: eine Handhabungseinrichtung in einer perspektivischen Ansicht;
- Fig. 4: die Handhabungseinrichtung mit einem Probenträger in einer weiteren perspektivischen Ansicht;
- Fig. 5: die Handhabungseinrichtung mit einer Abklatschprobenaufnahme in einer weiteren perspektivischen Ansicht;
- Fig. 6: die Handhabungseinrichtung in einer weiteren perspektivischen Ansicht;
- Fig. 7: die Handhabungseinrichtung während einer Probennahme in einer Seitenansicht;
- Fig. 8: die Handhabungseinrichtung während einer Probennahme in einer perspektivischen Ansicht;
- Fig. 9: eine Abklatschprobe auf einem Probenträger;
- Fig. 10: einen Probenträger mit Abklatschprobe; und
- Fig. 11: eine schematisch dargestellte Produktionsanlage mit einem geschlossenen oder getrennten Bereich in dem zwei Handhabungseinrichtungen angeordnet sind, die arbeitsteilig eine Probennahme durchführen können.

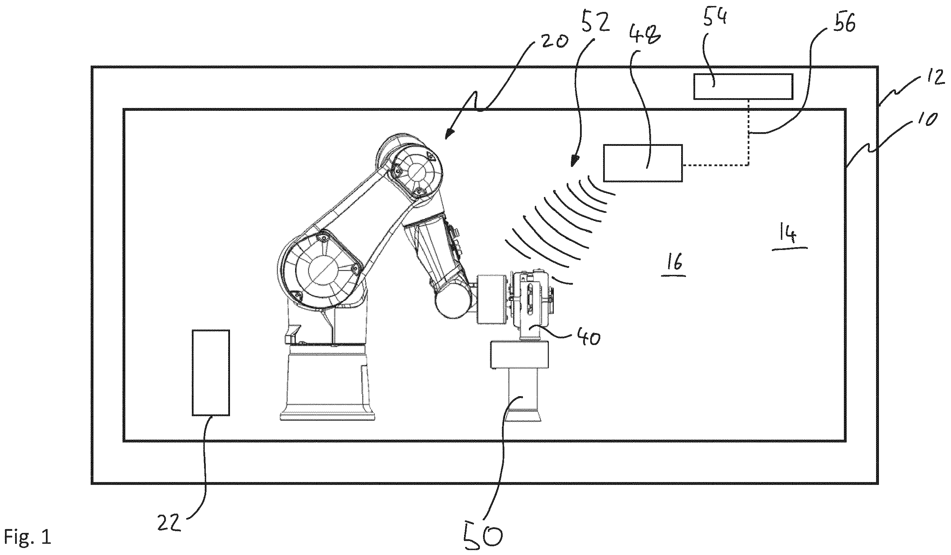

In Figur 1 ist eine pharmazeutische Produktionsanlage 12 mit einem Barrieresystem 10, das einen geschlossenen oder getrennten Bereich 14 umfasst, gezeigt. Das Barrieresystem 10 ist als Isolator 10 ausgebildet. In dem geschlossenen oder getrennten Bereich 14 des Isolators 10 ist eine sterile Umgebung 16 bereitgestellt.

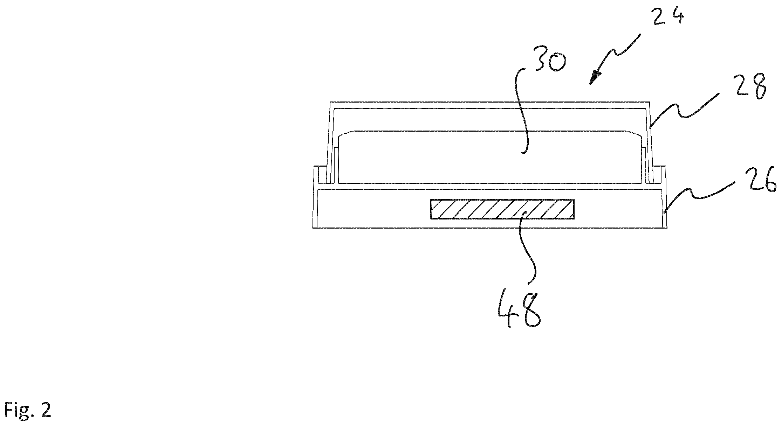

Innerhalb des geschlossenen oder getrennten Bereichs 14 ist eine Handhabungseinrichtung 20 und eine Abklatschprobenaufnahme 22 angeordnet. Die Abklatschprobenaufnahme 22 ist ausgebildet, um wenigstens eine Abklatschprobe 24, wie sie in Figur 2 illustriert ist bereitzustellen. Die gezeigte Abklatschprobenaufnahme 22 ist vorliegend zur Aufnahme mehrerer Abklatschproben 24 ausgebildet und die Abklatschproben 24 sind horizontal in der Abklatschprobenaufnahme 22 anordenbar. Wie in Figur 2 im Detail gezeigt, so umfasst die Abklatschprobe 24 einen Boden 26 und einen Deckel 28. Auf dem Boden 26 und durch den Deckel 28 verschlossen ist ein Nährmedium 30, typischerweise Agar, angeordnet. An dem Boden 26 ist weiter ein Identifizierungsmerkmals 48 angeordnet, das beispielsweise als QR-code (Data Matrix Code, oder Strichcode oder Information in einem RFID-Chip) ausgebildet sein kann. Das Identifizierungsmerkmal 48 befindet sich üblicherweise auf der äußeren Mantelfläche des Bodens 26. Der Deckel 28 ist abnehmbar, so dass eine Probenoberfläche 31 der Abklatschprobe 24 bzw. des Nährmediums 30 offenliegt.

Die Handhabungseinrichtung 20 ist ausgebildet, um die bereitgestellte Abklatschprobe 24 aus der Abklatschprobenaufnahme 22 zu entnehmen, zu öffnen und eine Probennahme auf einem Oberflächenabschnitt 42 in dem geschlossenen oder getrennten Bereich 14 durchzuführen. Der zu beprobende Oberflächenabschnitt 42 ist dabei auf einer Probenstelle 50 angeordnet, die ausgebildet ist, um reproduzierbare Probennahmen zu ermöglichen.

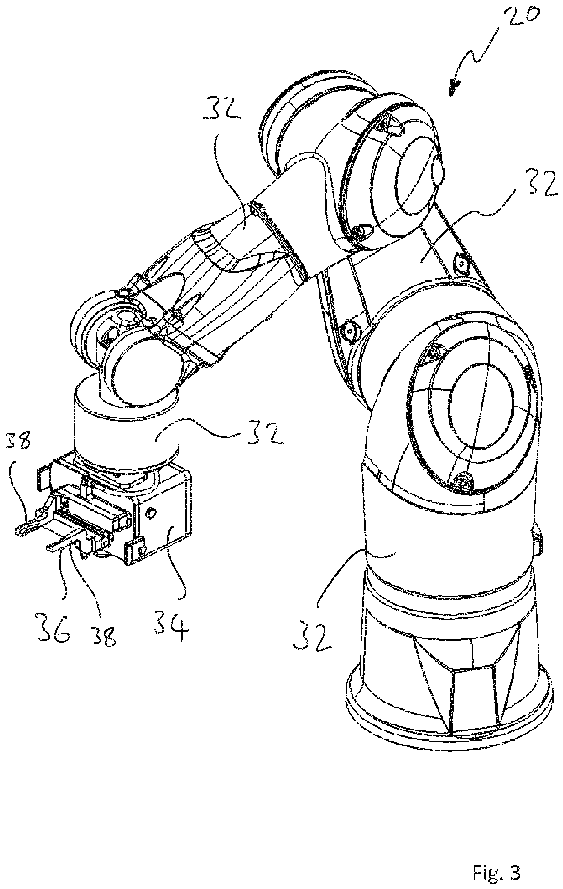

Die Handhabungseinrichtung 20 ist ausgebildet, um die Abklatschprobe 24 bei der Probennahme direkt greifen zu können. Hierzu weißt die Handhabungseinrichtung 20, wie in Fig. 3 gut erkennbar, einen Greifer 36 mit Greifbacken 38 auf. Der Greifer 36 ist an einem um 360° schwenkbaren Greiferglied 34 der Handhabungseinrichtung 20 angeordnet. Die Handhabungseinrichtung 20 weißt mehrere schwenkbar miteinander verbundene Glieder 32 auf, um den Greifer frei im Raum bewegen zu können.

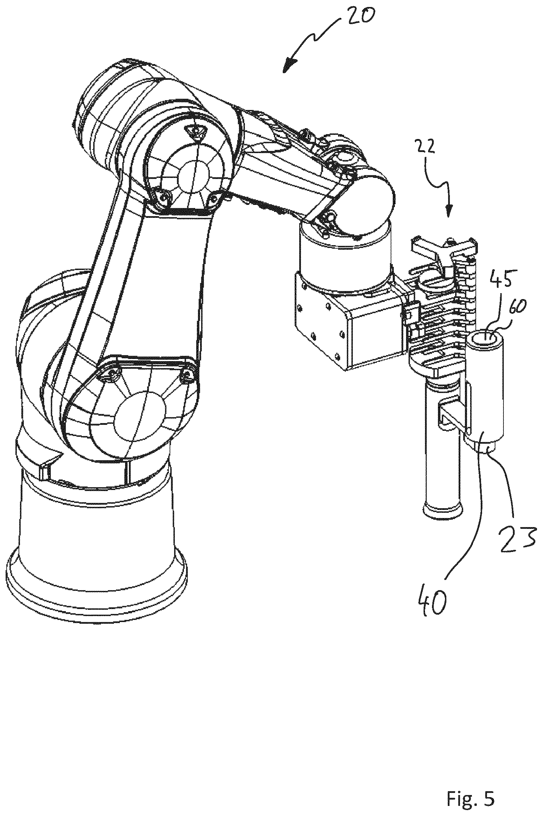

Die Abklatschprobenaufnahme 22 kann, wie in Figur 5 gezeigt, eine Halterung 23 für einen Probenträger 40 umfassen. Die Handhabungseinrichtung 20 kann über den Greifer 36 zwar die Abklatschprobe 24 direkt fassen, jedoch kann sie zur Probennahme auch den Probenträger 40 verwenden.

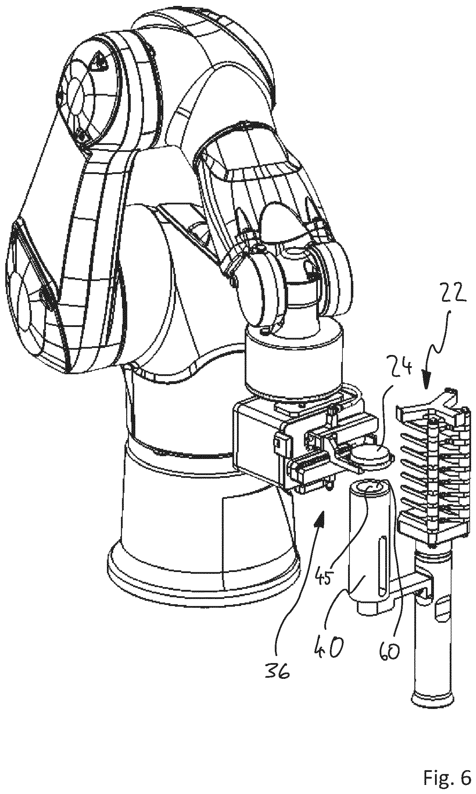

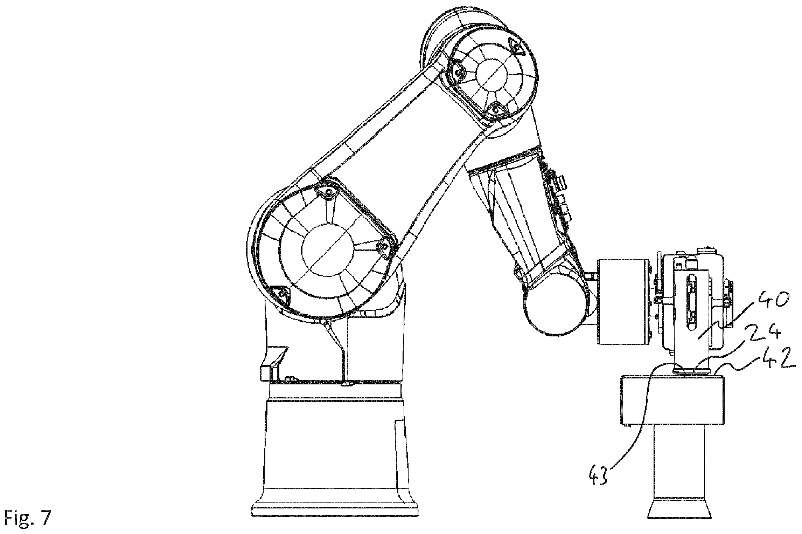

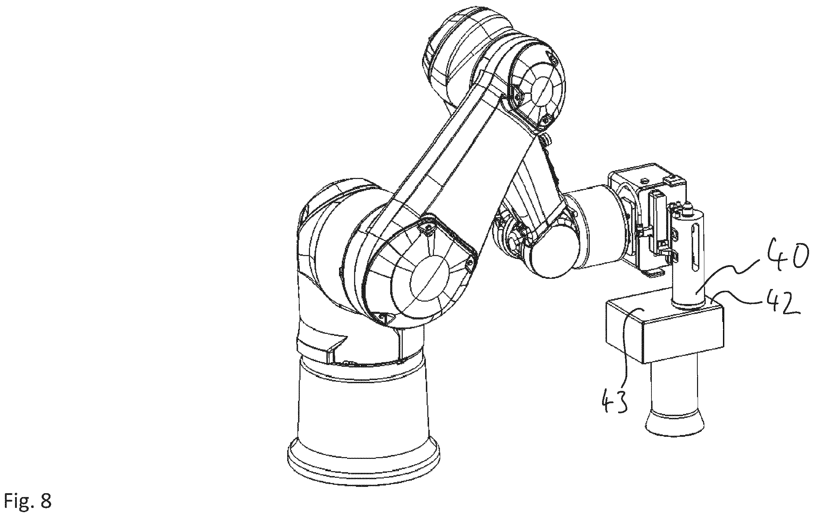

Die Handhabungseinrichtung 20 ist entsprechend ausgebildet, um die Abklatschprobe 24 nach dem Entnehmen aus der Abklatschprobenaufnahme 22 dem Probenträger 40 zuzuführen (siehe Figuren 5 und 6) und bei der Probennahme den Probenträger 40 zu bewegen (siehe Figur 7 und 8). Der Probenträger wird weiter unten noch weiter im Detail erläutert.

Das Barrieresystem 10 umfasst ein Erfassungsgerät 46, das ausgebildet ist, um ein Identifizierungsmerkmal 48 der Abklatschprobe 24 zu erfassen. Das Erfassungsgerät 46 umfasst oder ist typischerweise ein Strichcodeleser oder ein DataMatrixCode-Leser. Entsprechend kann das Identifizierungsmerkmal 48 ein entsprechender Code (Strichcode oder DataMatrixCode) auf einer Abklatschprobe 24 sein. Die Abklatschprobe 24 kann auch einen RFID-Chip mit dem Identifizierungsmerkmal 48 umfassen, so dass das Identifizierungsmerkmal 48 als Information auf dem RFID-Chip auslesbar bereitgestellt ist.

Über eine Datenverbindung 56, die kabelgebunden oder kabellos ausgebildet sein kann, ist das Erfassungsgerät 46 mit einer Datenverarbeitungsanlage 54 verbunden. Die Datenverarbeitungsanlage 54 kann die identifizierte Abklatschprobe 24 (erkannt durch ihr Identifizierungsmerkmal 48) der Zeit und/oder dem Ort einer jeweiligen Probennahme zuordnen, beispielsweise als einen entsprechenden Datensatz speichern. Der Erfassungsbereich 52 des Erfassungsgeräts 46 kann den Bewegungsbereich der Handhabungseinrichtung 20 umfassen oder die Abklatschprobe 24 kann mit ihrem Identifizierungsmerkmal 48 in den Erfassungsbereich 52 des Erfassungsgeräts 46 bewegt werden.

Da die zur Probennahme notwendigen Schritte in der Produktionsanlage 12 automatisiert durchgeführt werden kann das Barrieresystem 10 handschuheingrifffrei ausgebildet sein.

Erfindungsgemäß ist auch ein Verfahren zur automatisierten Probennahme in dem geschlossenen oder getrennten Bereich 14 des Barrieresystems 10. Dabei ist wie in der erfindungsgemäßen Produktionsanlage 12 in dem geschlossenen oder getrennten Bereich 14 wenigstens eine Handhabungseinrichtung 20 angeordnet ist. In dem geschlossenen oder getrennten Bereich 14 wird im Rahmen des Verfahrens die Abklatschprobe 24 bereitstellt und die Handhabungseinrichtung 20 erfasst (Figur 6) die bereitgestellte Abklatschprobe 24, öffnet diese, indem sie den Deckel 28 der Abklatschprobe 24 abnimmt und führt eine Probennahme auf einem Oberflächenabschnitt 42 in dem geschlossenen oder getrennten Bereich 14 durch.

In Figur 7 ist die Probennahme gezeigt, wobei die Abklatschprobe 24 hierzu mittels der Handhabungseinrichtung 20 auf dem Probenträger 40 platziert wurde.

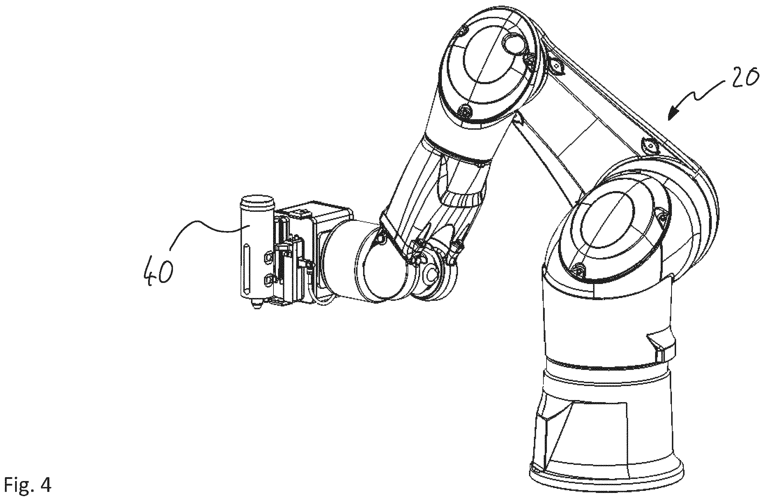

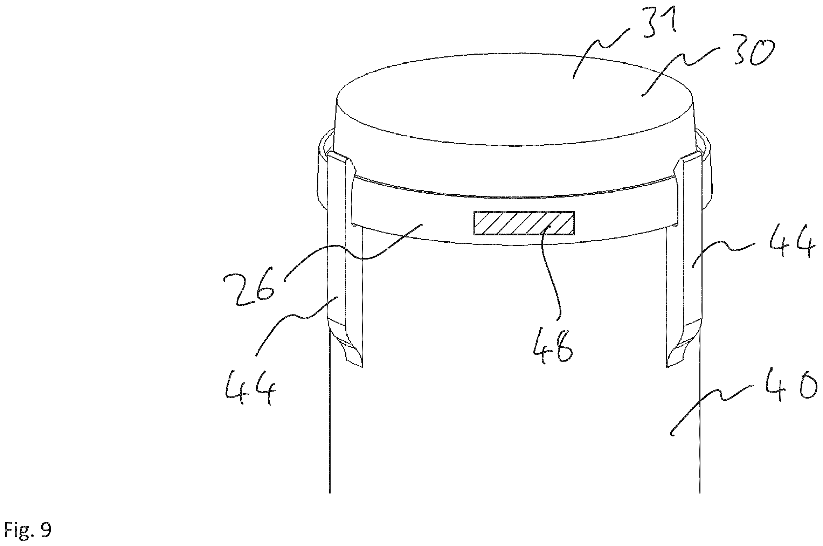

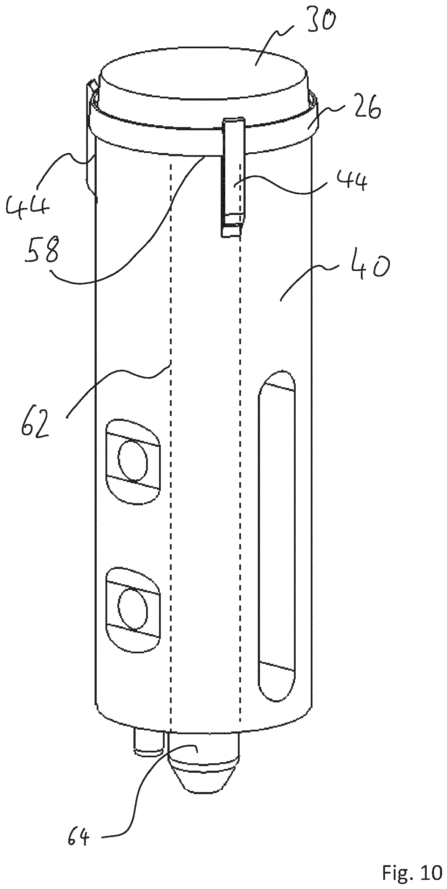

Der Probenträger 40 hält die Abklatschprobe 24 kraftschlüssig (Variante Figur 4 bis 8) und/oder formschlüssig (Variante Figuren 9 und 10). Der Formschluss wird über mehrere Befestigungsfinger 44, die in den Figuren 9 und 10 gezeigt sind erreicht. Die Befestigungsfinger 44 sind in Umfangsrichtung gleichmäßig verteilt um eine Anlagefläche 45 angeordnet. Die Anlagefläche 45 ist derart ausgebildet, dass die Abklatschprobe 24 flächig auf diese aufgelegt werden kann. Kraftschlüssig kann die Abklatschprobe 24 über eine Ansaugöffnung 58 oder eine adhäsive Oberfläche 60, die beispielsweise als mikrostrukturierte Polymerfolie ausgebildet sein kann, gehalten sein. Die Ansaugöffnung 58 ist über eine Druckleitung, die in dem Probenträger 40 verläuft mit einem Anschluss 64 für eine Vakuumversorgung verbunden.

Die Handhabungseinrichtung 20 öffnet die Abklatschprobe 24, die auf dem Probenträger 40 gehalten ist. Zur Probennahme greift sie den Probenträger 40 und bewegt den Probenträger 40 mit der an ihm gehaltenen Abklatschprobe 24 an den Oberflächenabschnitt 42 in dem geschlossenen oder getrennten Bereich 14 und führt die Probennahme durch Andrücken des Nährmediums an eine Oberfläche 43 des beprobten Oberflächenabschnitts 42 durch.

Die Handhabungseinrichtung 20 orientiert die Abklatschprobe 24 oder den Probenträger 40 mit der an ihm gehaltenen Abklatschprobe 24 bei der Probennahme derart, dass eine Probenoberfläche 31 der Abklatschprobe 24 parallel zur Oberfläche 43 des beprobten Oberflächenabschnitts 42 ausgerichtet ist und an diesen, insbesondere mit definiertem Druck und für eine definierte Dauer angedrückt wird.

Nach der Probennahme verschließt die Handhabungseinrichtung 20 die Abklatschprobe 24 durch Aufsetzen des Deckels wieder.

Die Handhabungseinrichtung 20 führt die Abklatschprobe 24 vor oder nach der Probennahme dem Erfassungsgerät 46 zu und das Erfassungsgerät 46 erfasst das Identifizierungsmerkmal 48 (siehe Figur 9) der Abklatschprobe 24. Nach der Probennahme und dem Erfassen des Identifizierungsmerkmals 48 führt die Handhabungseinrichtung 20 die Abklatschprobe 24 wieder der Abklatschprobenaufnahme 22 zu.

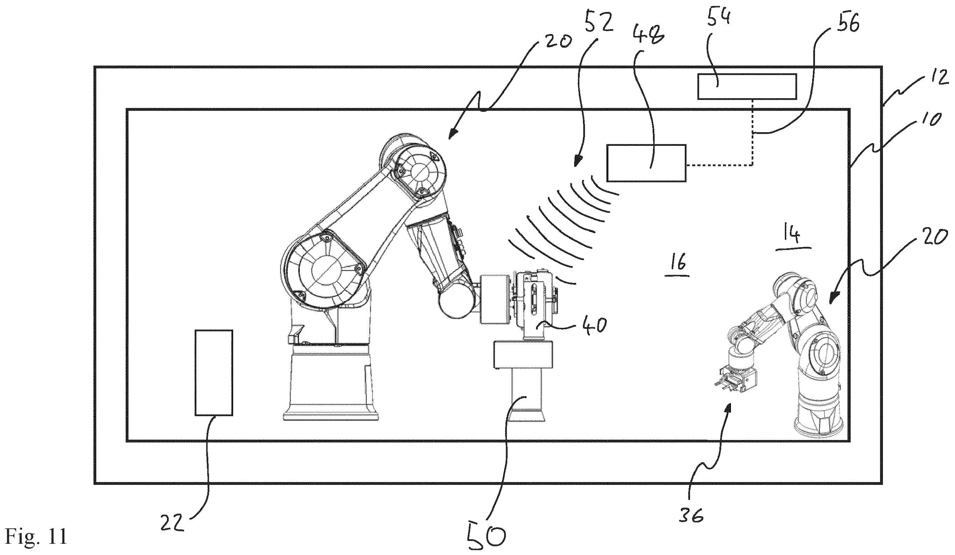

In Figur 11 ist ein Barrieresystem 10 mit zwei Handhabungseinrichtungen 20 illustriert. Die Handhabungseinrichtungen 20 arbeiten wechselweise und führen unterschiedliche Schritte, die zur Probennahme benötigt werden, durch. Die erste der beiden Handhabungseinrichtungen 20 erfasst die bereitgestellte Abklatschprobe 24. Die zweite der beiden Handhabungseinrichtungen 20 öffnet die bereitgestellte Abklatschprobe 24, nimmt also deren Deckel 28 ab die erste Handhabungseinrichtung 20 platziert die Abklatschprobe 24 auf einem Probenträger 40, die zweite Handhabungseinrichtung 20 greift den Probenträger 40 und führt mit der Abklatschprobe 24 eine Probennahme auf dem Oberflächenabschnitt 42 in dem geschlossenen oder getrennten Bereich 14 durch. Die erste Handhabungseinrichtung 20 verschließt die Abklatschprobe 24 nach der Probennahme durch Aufsetzen des Deckels 28 wieder. Danach platziert die zweite Handhabungseinrichtung 20 die Abklatschprobe 24 wieder in der Abklatschprobenaufnahme 22. Die einzelnen Schritte können auch von der jeweils anderen Handhabungseinrichtung 20 durchgeführt werden, jedoch führt jede der Handhabungseinrichtungen 20 wenigstens einen der Schritte des Verfahrens durch. Dies betrifft die erfindungsgemäße Verfahrensvariante mit zwei Handhabungseinrichtungen.

## Patentansprüche

1. Barrieresystem (10), insbesondere Isolator (10), einer, insbesondere pharmazeutischen, Produktionsanlage (12), mit einem geschlossenen oder getrennten Bereich (14), in dem eine sterile Umgebung (16) bereitgestellt wird, **dadurch gekennzeichnet, dass**
- in dem geschlossenen oder getrennten Bereich (14) eine Handhabungseinrichtung (20) und eine Abklatschprobenaufnahme (22) angeordnet sind,
- wobei die Abklatschprobenaufnahme (22) ausgebildet ist, um wenigstens eine Abklatschprobe (24) bereitzustellen, und
- wobei die Handhabungseinrichtung (20), ausgebildet ist, um automatisiert die bereitgestellte Abklatschprobe (24) aus der Abklatschprobenaufnahme (22) zu entnehmen, zu öffnen und automatisiert und eine Probennahme auf einem Oberflächenabschnitt (42) in dem geschlossenen oder getrennten Bereich (14) reproduzierbar durchzuführen.

2. Barrieresystem (10) nach Anspruch 1, **dadurch gekennzeichnet, dass** die Handhabungseinrichtung (20) ausgebildet ist, um die Abklatschprobe (24) bei der Probennahme direkt zu greifen.

3. Barrieresystem (10) nach Anspruch 1, **dadurch gekennzeichnet, dass** die Handhabungseinrichtung (20) ausgebildet ist, um die Abklatschprobe (24) nach dem Entnehmen einem Probenträger (40) zuzuführen und bei der Probennahme den Probenträger (40) zu bewegen.

4. Barrieresystem (10) nach einem der vorigen Ansprüchen, **dadurch gekennzeichnet, dass** die Handhabungseinrichtung (20) einen Greifer (36) mit zwei Greifbacken (38), die aufeinander zu und voneinander weg bewegbar sind, umfasst, insbesondere wobei der Greifer (36) an einem um wenigstens 90°, insbesondere um 180°, insbesondere um 360°, schwenkbaren Greiferglied (34) der Handhabungseinrichtung (20) angeordnet ist.

5. Barrieresystem (10) nach einem der vorigen Ansprüchen, **dadurch gekennzeichnet, dass** die Abklatschprobenaufnahme (22) eine Halterung (23) für den Probenträger (40) umfasst.

6. Barrieresystem (10) nach einem der vorigen Ansprüchen, **dadurch gekennzeichnet, dass** die Abklatschprobenaufnahme (22) zur Aufnahme mehrerer Abklatschproben (24) ausgebildet ist und die Abklatschproben (24) horizontal in der Abklatschprobenaufnahme (22) anordenbar sind.

7. Barrieresystem (10) nach einem der vorigen Ansprüchen, **dadurch gekennzeichnet, dass** es ein Erfassungsgerät (46) umfasst, das ausgebildet ist, um ein Identifizierungsmerkmals (48) der Abklatschprobe (24) zu erfassen, und insbesondere um die identifizierte Abklatschprobe (24) der Zeit und/oder dem Ort einer Probennahme zuzuordnen.

8. Barrieresystem (10) nach dem vorigen Anspruch, **dadurch gekennzeichnet, dass** das Erfassungsgerät (46) einen Strichcodeleser, einen DataMatrixCode-Leser, einen Klarschriftcode-Leser oder RFID-Lesegerät umfasst, der ausgebildet ist, um einen entsprechenden Code oder einer Information auf einer Abklatschprobe (24) oder einem Datenträger, an der Abklatschprobe (24), insbesondere einem RFID-Chip, zu lesen.

9. Barrieresystem (10) nach einem der vorigen beiden Ansprüche, **dadurch gekennzeichnet, dass** das Erfassungsgerät (46) ausgebildet ist, um den Ort und/oder den Zeitpunkt einer Probenentnahme zu erfassen, insbesondere wobei das Barrieresystem (10) eine Datenverarbeitungsanlage (54) umfasst, die eingerichtet ist, um den Ort und/oder den Zeitpunkt der Probenentnahme in einem Datensatz dem Identifizierungsmerkmal (48) der Abklatschprobe (24) zuzuordnen.

10. Barrieresystem (10) nach einem der vorigen Ansprüche, **dadurch gekennzeichnet, dass** das das Barrieresystem (10) handschuheingrifffrei ausgebildet ist.

11. Verfahren zur automatisierten Probennahme in einem geschlossenen oder getrennten Bereich (14) eines Barrieresystems (10), insbesondere Isolator (10), einer, insbesondere pharmazeutischen, Produktionsanlage (12), wobei in dem geschlossenen oder getrennten Bereich (14) eine sterile Umgebung (16) bereitgestellt ist, **dadurch gekennzeichnet, dass**
- in dem geschlossenen oder getrennten Bereich (14) wenigstens eine Handhabungseinrichtung (20) angeordnet ist,
- in dem geschlossenen oder getrennten Bereich (14) eine Abklatschprobe (24) bereitstellt ist, und
- die Handhabungseinrichtung (20) die bereitgestellte Abklatschprobe (24) erfasst, öffnet und eine Probennahme auf einem Oberflächenabschnitt (42), der insbesondere an einem statischen oder beweglichen Element angeordnet ist, in dem geschlossenen oder getrennten Bereich (14) durchführt.

12. Verfahren nach dem vorigen Anspruch, **dadurch gekennzeichnet, dass** die Handhabungseinrichtung (20) die Abklatschprobe (24) nach dem Erfassen auf einem Probenträger (40) platziert, der die Abklatschprobe (24), insbesondere kraft- und/oder formschlüssig, hält, die Abklatschprobe (24) öffnet, und zur Probennahme den Probenträger (40) greift und den Probenträger (40) mit der an ihm gehaltenen Abklatschprobe (24) an einen Oberflächenabschnitt (42) in dem geschlossenen oder getrennten Bereich (14) bewegt und die Probennahme durchführt.

13. Verfahren nach einem der vorigen Ansprüche, **dadurch gekennzeichnet, dass** die Handhabungseinrichtung (20) bei der Probennahme die Abklatschprobe (24) oder den Probenträger (40) mit der an ihm gehaltenen Abklatschprobe (24) derart orientiert, dass eine Probenoberfläche (31) der Abklatschprobe (24) parallel zur Oberfläche (43) des beprobten Oberflächenabschnitts (42) ausgerichtet ist und an diesen, insbesondere mit definiertem Druck und für eine definierte Dauer angedrückt wird.

14. Verfahren nach einem der vorigen Ansprüche, **dadurch gekennzeichnet, dass** die Handhabungseinrichtung (20) nach der Probennahme die Abklatschprobe (24) wieder verschließt.

15. Verfahren nach einem der vorigen Ansprüche, **dadurch gekennzeichnet, dass** die Handhabungseinrichtung (20) die Abklatschprobe (24) vor oder nach der Probennahme einem Erfassungsgerät (46) zuführt und das Erfassungsgerät (46) ein Identifizierungsmerkmal der Abklatschprobe (24) erfasst, insbesondere wobei das Identifizierungsmerkmal (48) einer Probennahme, insbesondere deren Zeit und/oder Ort zugeordnet wird.

16. Verfahren nach einem der vorigen Ansprüche, **dadurch gekennzeichnet, dass** die Handhabungseinrichtung (20) die Abklatschprobe (24) vor der Probennahme aus einer Abklatschprobenaufnahme (22) entnimmt und/oder nach der Probennahme einer Abklatschprobenaufnahme (22) zuführt.

17. Verfahren zur automatisierten Probennahme in einem geschlossenen oder getrennten Bereich (14) eines Barrieresystems (10), insbesondere Isolator (10), einer, insbesondere pharmazeutischen, Produktionsanlage (12), wobei in dem geschlossenen oder getrennten Bereich (14) eine sterile Umgebung (16) bereitgestellt ist, **dadurch gekennzeichnet, dass**
- in dem geschlossenen oder getrennten Bereich (14) wenigstens zwei Handhabungseinrichtung (20) angeordnet sind,
- in dem geschlossenen oder getrennten Bereich eine Abklatschprobe (24) bereitstellt ist, und
- wobei das Verfahren die Schritte umfasst:
- eine der Handhabungseinrichtungen (20) erfasst die bereitgestellte Abklatschprobe (24),
- eine der Handhabungseinrichtungen (20) öffnet die bereitgestellte Abklatschprobe (24),
- eine der Handhabungseinrichtung (20) führt mit der Abklatschprobe (24) eine Probennahme auf einem Oberflächenabschnitt (42) in dem geschlossenen oder getrennten Bereich (14) durch, wobei das Verfahren optional einen oder mehrere der nachfolgenden Schritte umfasst:
- Verschließen der Abklatschprobe (24) nach der Probennahme,
- Platzieren der Abklatschprobe (24) nach dem Erfassen auf einem Probenträger (40), der die Abklatschprobe (24), insbesondere kraft- und/oder formschlüssig, hält,
- den Probenträger (40) zur Probennahme greifen und den Probenträger (40) mit der an ihm gehaltenen Abklatschprobe (24) an einen Oberflächenabschnitt (42) in dem geschlossenen oder getrennten Bereich (14) bewegen und nachfolgend die Probennahme durchführen, wobei jede der Handhabungseinrichtungen (20) wenigstens einen der Schritte des Verfahrens durchführt.

18. Verfahren nach einem der vorigen Ansprüche 11 bis 16 oder 17, **dadurch gekennzeichnet, dass** nach einem ersten Produktionsintervall eine erste Probennahme mit ersten Probennahmeparametern durchgeführt wird und dass nach einem zweiten Produktionsintervall eine zweite Probennahme mit zweiten Probennahmeparametern durchgeführt wird, wobei die ersten und zweiten Probennahmeparameter wenigstens einen, insbesondere mehrere, identische Probennahmeparameter umfassen, insbesondere wobei die Probennahmeparameter identisch sind, insbesondere wobei die Probennahmeparameter einen oder mehrere der folgenden Parameter umfassen: Anpressdruck der Probe, Anpressdauer der Probe, Ort der Probennahme.
